# EUROPEAN PATENT APPLICATION

(11) **EP 4 595 766 A1**
(43) Date of publication of application: **06.08.2025**
(21) Application number: 24155647.1
(22) Date of filing: 03.02.2024
(51) Int. Cl.: A23G 4/12, A23G 4/06, A23G 4/08, A23G 4/10, A23L 33/105, A61K 9/68

(54) **DIETARY SUPPLEMENT IN THE FORM OF A CHEWING GUM FOR A COMPLEX EFFECT ON APPETITE CONTROL, WEIGHT REDUCTION AND ENERGY INCREASE**

(71) Applicant: Borisova-Dimitrova, Yoniela, 1510 Sofia (BG)
(72) Inventor: Borisova-Dimitrova, Yoniela, 1510 Sofia (BG)

(57) **Abstract**

Very often people give up on the fight against the extra pounds on their body. They can't resist the delicacies in the store or the pastry shop and start gaining more and more pounds, which demotivates them to make any effort to lose weight.

The chewing gum that I offer is a combination of components that have a complex positive effect when used - reducing and controlling appetite, regulating the blood sugar levels, contributing to the acceleration of metabolism, helping to reduce the feeling of fatigue, exhaustion and lethargy.

It also contributes to increasing physical endurance and increasing energy, therefore enhancing the process of losing weight and burning excess fat. It also has a favorable effect on concentration, memory and mental activity.

In order to have a good and long-lasting effect when taking the gum for weight reduction, the user must combine the nutritional supplement-gum with light sports and moderate food intake.

It is recommended to take two chewing gums a day by people who are 18+ years old.

For the consumption of the chewing gum, pregnant and nursing mothers should consult a doctor.

## Description

The present patent refers to a composition , which includes: **Acetyl L-CARNITINE**, which is the biologically active and highly digestible form of the amino acid L-CARNITINE in its (natural) form. It's a part of the metabolism and helps to convert fat into energy, which in turn leads to a decrease in body weight. It has a favorable effect on concentration, memory and mental activity.

**Chromium Picolinate** lowers blood sugar levels. It helps regulate the carbohydrate metabolism and thus reduces the desire for sweet foods. Helps with weight reduction. It contributes to the maintenance of normal blood sugar levels and optimizes the action of insulin. Supports the normal metabolism of macronutrients.

**GREEN TEA** contributes to the process of weight loss and the burning of subcutaneous fat, the high levels of antioxidants in green tea have a favorable effect on weight loss. GREEN TEA regulates cholesterol, prevents the development of type 2 diabetes, accelerates the natural burning of body fat and improves brain functions.

In addition to the components mentioned in the content, the chewing gum of the patent contains natural sweeteners, which are harmless substitutes for sugar. They do not increase the blood sugar level and contribute to cleansing the body of toxins and slags, improve digestion and metabolism. The sweeteners are such as the following ones - Stevia, Xylitol, Erythritol, Sorbitol, Manitol, Siraitia Grosvenorii The chewing gum contains natural aromas and flavors that are harmless, a natural gum (rubber) base, emulsifiers, softeners, resinous compounds to make it pleasant and harmless to use.

### Prior State of the Patent

The enticing appearance of the food products that constantly flood the market stimulate the desire to consume them, and work obligations and the lack of free time for sports and walks hinder people's movement, which contributes to the slowing down of metabolism and rapid weight gain.

The purpose of the chewing gum food supplement is a complex effect of powerful ingredients to reduce the desire for carbohydrates, increase the body's energy, which is a prerequisite for the desire for sports and help with weight reducement

### Technical nature of the patent

Very often people give up in the fight against the extra pounds. They can't resist the delicacies on the shelves of the store or the pastry shop and start gaining more and more weight, which demotivates them to make any effort to lose weight.

The chewing gum that I offer is a combination of components that have a complex effect - they limit the desire (appetite) for sweets, accelerate metabolism, regulate cholesterol, prevent the development of type 2 diabetes, stimulate the production of enzymes that increase the burning of calories, leading to a decrease in weight and increase in energy and physical endurance, it also improves the brain function.

In order to have a good and long-lasting effect when taking the weight loss gum, the user must combine the gum with light sports and moderate food intake.

According to the present patent, the composition of the chewing gum contains the following components:
**Acetyl L-CARNITINE:** It is preferred that the composition of Acetyl L-CARNITINE in the gum of the present patent be from about 2 wt. %, up to about 15 wt.% by weight content of the total weight of the chewing gum composition.

**CHROMIUM Picolinate:** It is preferred that the composition of CHROMIUM Picolinate in the gum of the present patent be from about 2 wt. %, up to about 16 wt.% by weight content of the total weight of the chewing gum composition.

**GREEN TEA** It is preferred that the composition of GREEN TEA in the gum of the present patent be from about 2 wt. %, up to about 15 wt.% by weight content of the total weight of the chewing gum composition. GREEN TEA can be in the form of a dry leaf extract or in the form of a tincture created from ethanol and green tea leaves 1:5 equivalent to 250mg. GREEN TEA Ethanol 66% vol.

**Natural sweeteners.** In order to increase the taste properties of the gum, whilst not contain calories, natural sweeteners were used for this patent, some of which are sweeter than sugar. According to the present patent, the chewing gum composition contains a pharmaceutically acceptable natural sweetener such as Stevia and/or Xylitol and/or Erythritol and/or Sorbitol and/or Mannitol and/or Siraitia Grosvenorii which imparts volume, sweetness and a pleasant taste which in amounts and in range, from about 5 wt.% to 60 wt.% of the total weight of the chewing gum. Stevia regulates blood sugar levels
**Chewing gum base.** According to the present patent, the composition of the chewing gum contains a base for chewing gum, which is created from chewing substances, preferably of plant origin such as Chicle, Mastix. The gum base contains a humectant (glycerin) to form a stable whole gum, which is combined with harmless plasticizers such as carnauba wax, glycerin and components known in the chewing gum manufacturing industry. The gum base in the gum is from about 19% by weight to about 70% by weight.

Typical examples of the ingredients found in the base of the chewing gum are chewing substances of vegetable origin (Chicle, Mastix and others of natural origin), which can be used together or separately, and can also be replaced with an innovative harmless analogue of the base of chewing gum , which would be harmless for the human body.

**Flavoring of the chewing gum.** Regarding the flavoring agent included in the composition of the chewing gum, combined and/or individual flavors were used in dry and/or liquid form, fruit essences, essential oils of herbs - mint (menthol), clove, laurel, anise, cinnamon, eucalyptus.

The chewing gum composition which includes the said flavoring agents may be sweet, slightly sour, minty, fruity or flavored, according to said flavoring agents used in an amount of about 0.2 wt. % to about 10 wt.% based on the content of the final product to make the chewing gum pleasant to use.

The chewing gum obtained from a composition according to the present patent may have a glossy coating, which may be colored or white, depending on the flavoring and flavoring agents used.

The present product also relates to a package containing a chewing gum composition according to the present patent.

According to the patent of a food supplement in the form of chewing gum, it contains weight units in %. The whole mass for 1 piece of chewing gum is 1.5g; 1500mg: 100 =15mg= 1%

### Example implementation of the utility model

The components presented in the table are a specific implementation of the patent, with the aim of having a complex effect when used - reducing and controlling appetite, regulating blood sugar levels, helping to accelerate metabolism. It helps to reduce the feeling of fatigue, exhaustion and lethargy. Contributes to increasing physical endurance and increasing energy. It enhances the process of losing weight and burning excess fat. It has a favorable effect on concentration, memory and mental activity.

The contents of the chewing gum gradually break down in the mouth and enter the body through the saliva. Accumulating, the desire for sweet foods decreases, and the weight gradually begins to decrease. The combination of light sports and a moderate diet enhance the effect of chewing gum.

The list of ingredients in this patent is presented in percentages.

Contents of the chewing gum food supplement:

| Ingredients | Contents in % |
|---|---|
| Acetil L-CARNITINE | 8% |
| Chromium Picolinate | 5% |
| GREEN TEA | 4% |
| Chewing Gum Base | 39,5% |
| Chewing gum sweetener (liquid and powder) | 40 % |
| Chewing gum flavor (liquid and powder) | 3% |
| Others (glycerin, lim.k-na, tapioca or talc) | 0,5% |
| Total | 100% |

### Recommendations:

It is recommended for the chewing gum to be consumed by people over 18 years of age. The recommended daily amount is two gums per day.

Pregnant and nursing mothers should consult a doctor before consuming the product.

## Claims

1. A food supplement composition in the form of chewing gum, containing Acetyl L-CARNITINE, Chromium Picolinate, GREEN TEA (It can be in the form of a dry leaf extract or in the form of a tincture created from ethanol and leaves from green tea), natural and harmless sweeteners such as Stevia and/or Xylitol and/or Erythritol and/or Sorbitol and/or Mannitol and/or Siraitia Grosvenorii, gum base - preferably of plant origin such as Chicle, Mastix or other gum , which is authorized for use in the production of chewing gum, and in order to form a stable complete chewing gum, the base is combined with harmless plasticizers such as carnauba wax, glycerin and components known in the production of chewing gum, natural flavors in dry and/or liquid form.

2. Composition of a food additive in the form of chewing gum, according to claim one, characterized with the content of Acetyl L-CARNITINE in the chewing gum of the present patent being about 2 wt. %, up to about 15 wt.% by weight content of the total weight of the chewing gum composition.

3. Composition of a food supplement in the form of chewing gum, according to claim one, characterized with the content of CHROMIUM Picolinate in the composition of the chewing gum of the present patent being about 2 wt. %, up to about 16 wt.% by weight content of the total weight of the chewing gum composition

4. Composition of a food supplement in the form of chewing gum, according to claim one, characterized with the content of GREEN TEA in the chewing gum of the present patent being about 2 wt. %, up to about 15 wt.% by weight content of the total weight of the chewing gum composition. It can be in the form of a dry leaf extract or in the form of a tincture created from ethanol and green tea leaves 1:5 equivalent to 250mg. GREEN TEA Ethanol 66% vol.

5. Composition of a food supplement in the form of chewing gum, according to claim one **characterized by** the content of the Natural Sweeteners Stevia and/or Xylitol and/or Erythritol and/or Sorbitol and/or Mannitol and/or Siraitia Grosvenorii, of the present patent being about 5 wt.% to 60 wt.% of the total weight of the chewing gum. They can be used only in a liquid state or only in a dry state, but they can also be used combined in a liquid and dry state in different percentages until a sweet pleasant taste is obtained.

6. A food supplement composition in the form of a chewing gum according to claim one **characterized by** the content of the Gum Base of the present patent being from about 19 wt.% to about 70 wt.% of the total weight of the gum.

7. Composition of a food additive in the form of chewing gum, according to claim one, **characterized by** the content of the Flavoring of the chewing gum of the present patent being about 0.2 wt. % to about 10 wt.% of the total weight of the chewing gum.

## Amended claims

### Amended claims in accordance with Rule 137(2) EPC.

1. A food supplement composition in the form of chewing gum, aiming for a complex effect over the body - reducing the desire for food containing sugar, controlling the appetite, supporting the brain activity , reducing weight, heightening the energy levels. Contents consist of Acetyl L-CARNITINE, Chromium Picolinate, GREEN TEA (It can be in the form of a dry leaf extract or in the form of a tincture created from ethanol and leaves from green tea), natural and harmless sweeteners such as Stevia and/or Xylitol and/or Erythritol and/or Sorbitol and/or Mannitol and/or Siraitia Grosvenorii, gum base - preferably of plant origin such as Chicle, Mastix or other gum, which is authorized for use in the production of chewing gum, and in order to form a stable complete chewing gum, the base is combined with harmless plasticizers such as carnauba wax, glycerin and components known in the production of chewing gum, natural flavors in dry and/or liquid form.

2. Composition of a food additive in the form of chewing gum, according to claim one, characterized with the content of Acetyl L-CARNITINE in the chewing gum of the present patent being about 2 wt. %, up to about 15 wt.% by weight content of the total weight of the chewing gum composition. Acetyl L-CARNITINE plays an important role in the metabolism as it supports the process of fat burning, which reduces the weight, it supports the brain function.

3. Composition of a food supplement in the form of chewing gum, according to claim one, characterized with the content of CHROMIUM Picolinate in the composition of the chewing gum of the present patent being about 2 wt. %, up to about 16 wt.% by weight content of the total weight of the chewing gum composition.
Chromium Picolinate regulates the carbohydrate metabolism and so it reduces the desire for sweet food. It helps in reducing the weight and helps keeping the blood sugar levels normalized. It also optimizes the way insulin functions.

4. Composition of a food supplement in the form of chewing gum, according to claim one, characterized with the content of GREEN TEA in the chewing gum of the present patent being about 2 wt. %, up to about 15 wt.% by weight content of the total weight of the chewing gum composition. It can be in the form of a dry leaf extract or in the form of a tincture created from ethanol and green tea leaves 1:5 equivalent to 250mg. GREEN TEA Ethanol 66% vol.
Green tea naturally creates the feeling for awakeness and energy in the body. It helps with the process of weight loss and fat burning. It supports the body with the antioxidant shield it creates.
The three ingredients in the gum create a mighty formula , that has many positive impacts on the body, one of which would be weight reduction.

5. Composition of a food supplement in the form of chewing gum, according to claim one **characterized by** the content of the Natural Sweeteners Stevia and/or Xylitol and/or Erythritol and/or Sorbitol and/or Mannitol and/or Siraitia Grosvenorii, of the present patent being about 5 wt.% to 60 wt.% of the total weight of the chewing gum. They can be used only in a liquid state or only in a dry state, but they can also be used combined in a liquid and dry state in different percentages until a sweet pleasant taste is obtained.
For a perfect flavor, the natural sweeteners are being combined in a way that makes for the most pleasurable taste in the chewing gum for the customers.

6. A food supplement composition in the form of a chewing gum according to claim one **characterized by** the content of the Gum Base of the present patent being from about 19 wt.% to about 70 wt.% of the total weight of the gum - enough for the gum to have a nice soft chewing feel to it.

7. Composition of a food additive in the form of chewing gum, according to claim one, **characterized by** the content of the Flavoring of the chewing gum of the present patent being about 0.2 wt. % to about 10 wt.% of the total weight of the chewing gum.
The aroma of the chewing gum meant for weight loss should stimulate the feeling for freshness, lower the appetite, being pleasant and refreshing at the same time.
The mint aroma helps decrease the desire for food consumption and the feeling for hunger.
The citrus aromas of lemon, lime, grapefruit add to the refreshing and stimulating scent, that can help speed up the metabolism and feeling of energy.
